# DEMANDE DE BREVET EUROPEEN

(11) **EP 1 323 414 A2**
(43) Date de publication de la demande: **02.07.2003**
(21) Numéro de dépôt: 02380235.8
(22) Date de dépôt: 15.11.2002
(51) Int. Cl.: A61K 9/00

(54) **Améliorations introduites dans des produits destinés à la consommation humaine ou animale**

(30) Priorité: 16.11.2001 ES 200102540
(71) Demandeur: High-Tech Product, S.L., 43001 Tarragona (ES)
(72) Inventeur: Balufo Sanchez, Juan, 08004 Barcelona (ES); Tico Grau, José Ramon, 08004 Barcelona (ES)
(74) Mandataire: Urizar Anasagasti, Jesus Maria

(57) **Abrégé**

Les améliorations faisant l'objet de l'invention envisagent que les produits en question puissent se présenter sous forme de granulés ou de particules constituées de matières actives seules, ou associées à d'autres, ou en combinaison à des excipients, et d'un agent hydrophobe substantiellement insoluble dans l'eau, en état solide, avec un point de fusion compris entre 10 et 80 degrés centigrades, en maintenant la matière active isolée et l'empêchant de commencer son activité tant que n'a pas eu lieu la fusion de l'agent hydrophobe.

## Description

### OBJET DE L'INVENTION

La présente invention concerne les améliorations introduites dans les produits destinés à la consommation humaine ou animale, ayant une activité pharmacologique ou pas, et n'étant pas stables à l'air, à la lumière ou à l'humidité, ou présentant des caractéristiques organoleptiques désagréables; ces produits étant constitués d'une ou de plusieurs matières actives, associées ou pas à des excipients, pouvant s'agir de : antibiotiques (enrofloxacine, amoxiciline, ...), sulfamides, mono et disaccharides, sels minéraux, acides organiques, acides inorganiques et leurs sels, protéines, enzymes, aminoacides, micro-organismes atténués (vaccins), vitamines et provitamines, extraits naturels, produits d'origine végétale (algues, etc.) et animale.

Les améliorations de cette invention consistent à pourvoir les produits mentionnés d'un traitement de pelliculage avec un agent hydrophobe, en les transformant en produits stables au milieu ambiant (air, lumière et humidité) ou en camouflant leur possible mauvais goût, odeur ou couleur.

Cet agent hydrophobe substantiellement insoluble dans l'eau en état solide, peut être incorporé à l'aide de froid, ou de vide, ou de chaleur o de forces de cisaillement avec un point de fusion compris entre les 10 et les 80 degrés centigrades.

### ANTÉCÉDENTS DE L'INVENTION

Les antibiotiques sont des matières actives qui sont normalement instables au milieu ambiant. Nombre d'eux sont altérés par la présence de la lumière, l'air et l'humidité; c'est-à-dire qu'il se produit une perte évidente de leur activité antibactérienne.

Dans d'autres cas, comme dans l'enrofloxacine et l'aritromicine, ils présentent un goût très désagréable. Ces caractéristiques ne permettent pas de l'administrer tel quel, par exemple, aux porcs ou autres animaux.

Ces problèmes apparaissent aussi pour la plupart des sulfamides.

Un autre type de matières premières qui s'altèrent avec l'humidité ambiante sont les sucres, comme le saccharose. Celui-ci, quand il s'humidifie, perd des propriétés organoleptiques, ce qui rend son emploi postérieur très délicat. Actuellement, il faut leur ajouter d'autres excipients permettant de faciliter leur maniement.

Les vitamines, provitamines et leurs dérivés (sels, esters) sont généralement hygroscopiques et par conséquent elles subissent rapidement une perte d'activité. De même, elles subissent normalement des réactions de dégradation par photosensibilité, par la présence d'oxygène et par les changements de chaleur. Des exemples de ces altérations sont la vitamine C ou l'acide ascorbique, la vitamine B6 et la vitamine B12.

De même, les protéines, enzymes et aminoacides s'altèrent normalement à cause de l'humidité, l'air et la lumière. De plus, certains, comme la métionine sentent très mal, ce qui la rend difficile à administrer.

De même, des produits comme les micro-organismes vivants, atténués et les levures se décomposent en contact avec l'air et avec l'humidité, et il est nécessaire, par conséquent, de les protéger pour conserver leur activité.

Les extraits d'origine naturelle, les produits d'origine végétale et animale sont généralement très hygroscopiques ce qui, en plus de diminuer leur richesse ou activité, les rend difficiles à manipuler.

Les sels minéraux, comme par exemple le sulfate de zinc, le sulfate de fer et le sulfate de cuivre présentent aussi des problèmes d'altération au milieu ambiant et de mauvaises caractéristiques organoleptiques.

Les acides organiques et inorganiques et leurs sels et dérivés sont utilisés dans l'alimentation humaine et animale mais ils ne peuvent pas être employés à cause des mêmes problèmes déjà mentionnés. Comme ils sont normalement administrés seuls ou associés et mélangés à d'autres matières premières et des excipients, il faut les protéger pour éviter des incompatibilités entre eux et d'eux avec d'autres matières premières ou excipients.

Actuellement, pour éviter ce genre de problèmes, on réalise des enrobages, en général au moyen de gélatines qui sont normalement d'origine animale, de sucres, maltodextrines, amidons, caoutchoucs végétaux et autres produits à grand poids moléculaire. Les procédés généralement employés sont la conservation, évaporation de solvant, lit fluide, extrusion centrifuge et rotogranulateur.

Ces traitements ou procédés d'enrobages appliqués actuellement, sont des procédés en général très coûteux et de production relativement faible.

### DESCRIPTION DE L'INVENTION

Pour résoudre cette problématique, on a introduit dans les produits mentionnés les améliorations faisant l'objet de l'invention, qui permettent à un prix relativement bas de les transformer en produits stables au milieu ambiant (air, lumière et humidité) et de camoufler leur mauvais goût, odeur ou couleur.

On a pensé, comme objet de cette invention, à une combinaison de procédés et d'excipients hydrophobes rendant stables au milieu ambiant et éliminant les caractéristiques organoleptiques indésirables des produits sus-mentionnés.

Conformément à l'invention, les améliorations appliquées aux produits constitués, par exemple, de particules ou de granulés contenant la matière ou matières premières actives, associées à des excipients, consistent à l'incorporation dans ceux-ci d'un agent hydrophobe qui, en état solide, est substantiellement insoluble dans l'eau et qui peut être appliqué à l'aide de froid, vide, chaleur, forces de cisaillement, transformation en nodules, frittage, compactage, agglomération, transformation en globules ou combinaisons des précédents; cet agent hydrophobe présentant un point de fusion compris entre les 10 et les 80 degrés centigrades.

Il a été prévu que l'agent hydrophobe incorporé au produit soit, en état solide, insoluble dans l'eau et soluble, miscible ou dispersable dans l'eau quand il est fondu.

Ladite matière hydrophobe maintient les caractéristiques de protection de la matière première active, empêchant sa dégradation, réaction et libération de ses caractéristiques organoleptiques entre les -20 et +75 degrés centigrades.

Une autre caractéristique de l'invention consiste à ce que la température de la matière première active, seule ou combinée à d'autres matières premières actives ou des excipients, est maintenue à une température inférieure à 50°C pendant l'incorporation du produit hydrophobe, afin d'empêcher que cette incorporation provoque une détérioration des caractéristiques des matières premières actives.

Conformément à l'invention, la matière première active constituée des particules ou granulés, peut être seule ou mélangée à des substances inertes en guise d'excipient ou combinée à d'autres matières premières actives, ou aux deux, tandis que l'agent hydrophobe, substantiellement insoluble dans l'eau en état solide, peut être constitué de produits d'origine végétale tels que : cires d'utilisation alimentaire, graisses alimentaires ou margarines, ou des préparations grasses comme les graisses inertes ou les acides gras, ou des mono ou di ou triglycérides d'acides gras non polymérisés à chaîne linéaire saturés et insaturés, estérifiés ou pas, présents dans les huiles et/ou graisses alimentaires, ou des mono ou di ou triglycérides d'acides gras estérifiés avec les acides organiques alimentaires estérifiés ou pas et leurs sels calciques, de sodium et potassiques ou les esters de glycérol, ou les esters de glycérol interestérifié avec des acides gras d'huile de ricin, ou des esters de sorbitan ou toute autre combinaison des précédents.

La quantité d'agent hydrophobe incorporé est comprise entre 5% et 50% du poids du produit final, les granulés ou particules de produit composées de la matière première active, avec ou sans excipients, et l'agent hydrophobe, présentant des dimensions comprises entre 50 microns et 10 millimètres.

La fusion ou dégradation de l'agent hydrophobe incorporé à la matière active est seulement possible au moyen de procédés enzymatiques ou d'un point ou séquence de points de température.

Moyennant les améliorations indiquées, consistant en l'incorporation de l'agent hydrophobe à la matière première active, combinée ou pas à des excipients, on obtient les avantages suivants :
a) Isoler du milieu extérieur, ou camoufler les mauvaises caractéristiques organoleptiques, les matières actives constituant les particules ou granulés, en maintenant intacte leur capacité de réaction ou activité pendant de longues périodes de temps. Cela permet la conservation de produits finis ou intermédiaires, dans des milieux secs ou saturés d'humidité et à des températures comprises entre -40°C et +75°C.
b) Cela permet de réduire considérablement les quantités de produit (réduire le dosage ou le surdosage) de ces matières premières actives employées habituellement puisque, la capacité totale de l'activité initiale étant maintenue, il n'est pas nécessaire d'utiliser une plus grande quantité de matière première active comme cela arrive avec les matières premières non stables à l'air, la lumière ou l'humidité utilisées actuellement et qui perdent progressivement ladite capacité de réaction ou activité, à cause des conditions physiques, mécaniques ou environnementales.
c) Cela permet de conserver la capacité de réaction ou activité initiale du produit composé de particules ou granulés obtenus par le traitement avec l'agent hydrophobe substantiellement insoluble en état solide, à température ambiante, pendant un temps maximum pour chaque produit actif et sans aucune restriction ni obligation hors de l'ordinaire en matière de manipulation, emballage, conditionnement et transport.
d) Cela permet de conserver les bonnes caractéristiques organoleptiques obtenues par le traitement avec l'agent hydrophobe substantiellement insoluble dans l'eau, en état solide, des particules ou granulés à température ambiante, pendant le temps maximum pour chaque produit et sans aucune restriction ni obligation hors de l'ordinaire en matière de manipulation, emballage, conditionnement et transport.
e) Ces produits actifs ultimes se présentent de préférence sous forme de particules ou granulés pour faciliter leur distribution homogène et régulière dans les applications auxquelles ils sont destinés et pour assurer que le traitement avec l'agent hydrophobe substantiellement insoluble dans l'eau, en état solide, maintient les matières premières actives qu'ils contiennent, protégées et conserve pendant de longues périodes de temps leur capacité de réaction et leur activité ou richesse initiale. Ces granulés ou particules peuvent avoir des dimensions différentes, se trouvant comprises de préférence entre 50 microns et 10 millimètres, bien qu'il existe aussi une possibilité de présentation du produit dans une plus grande taille dans le cas où la nécessité du marché l'imposerait.
f) Le traitement avec l'agent hydrophobe substantiellement insoluble dans l'eau, en état solide, peut représenter un poids compris entre 5% et 50% du poids total du produit final, la quantité pouvant varier en fonction de la technologie ou des procédés globaux employés.
g) Ledit traitement avec l'agent hydrophobe substantiellement insoluble dans l'eau, en état solide, empêche les réactions de dégradation et améliore les caractéristiques organoleptiques (couleur, odeur et goût) de la matière première active entre les -40 et -75 degrés centigrades, indépendamment du type de procédé ou de technologie employée pour obtenir la protection hydrophobe finale.
   L'utilisation de ces améliorations envisage que le point de fusion du pelliculage des particules ou granulés constitutifs du produit final soit celui qui résulte des points de fusion des produits hydrophobes utilisés en fonction de l'application à laquelle est destiné le produit final.
h) L'activité ou la richesse des particules ou granulés de la matière première active finale ou produit final peut s'activer ou se déclencher moyennant la fusion de l'agent hydrophobe substantiellement insoluble dans l'eau, en état solide. Ladite fusion peut être obtenue à un point ou séquence de points de température déterminés en fonction de l'application finale ou par effet enzymatique.

Les améliorations de cette invention permettent aussi la combinaison ou association de différentes matières premières incompatibles entre elles, dû à la protection fournie par le produit hydrophobe, dans les applications exigeant leur combinaison, tout en les préservant dans les conditions ambiantes déjà mentionnées et/ou avec des caractéristiques organoleptiques désagréables.

### DESCRIPTION DES FIGURES

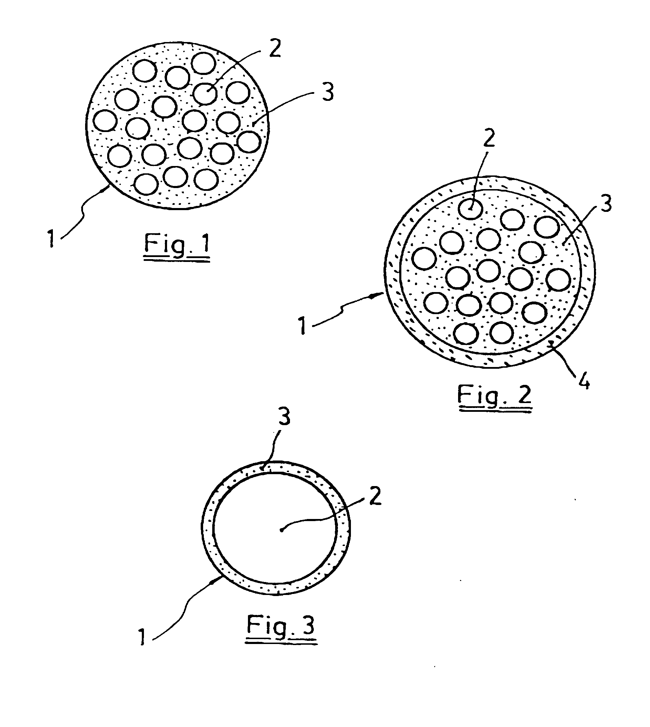

Pour compléter la description en cours et afin de faciliter la compréhension des caractéristiques du brevet faisant l'objet de cette invention, le présent mémoire descriptif est accompagné d'un jeu de dessins qui représentent, à titre d'illustration et sans limitation, ce qui suit :
- Chacune des figures 1, 2 et 3 montre des coupes transversales, à grande échelle, des différentes réalisations d'un des granulés ou particules constitutives du produit final avec les améliorations faisant l'objet de l'invention.

### RÉALISATION PRÉFÉRENTIELLE DE L'INVENTION

Comme on peut l'observer dans les figures susmentionnées, les particules ou granulés, faisant l'objet de cette invention, sont composés d'un produit ou de produits ayant une activité pharmacologique ou pas, non stables à l'air, à la lumière, à l'humidité ou ayant des caractéristiques organoleptiques désagréables et qui, à partir d'un traitement avec un agent hydrophobe substantiellement insoluble dans l'eau, en état solide, se transforment en produits stables dans le milieu ambiant (air, lumière et humidité) ou bien, dans le cas de matières actives ayant de mauvaises caractéristiques organoleptiques, leur mauvais goût, odeur ou couleur est camouflé.

Plus précisément, dans l'exemple de réalisation montré dans la figure 1, la particule ou granulé (1) est composé de matières premières actives (2) de même ou différente nature, combinées ou pas à des excipients, et d'un agent hydrophobe (3), la particule ou granulé (1) présentant une structure à la manière de systèmes matriciels ou de matrices lipidiques.

Moyennant l'incorporation de l'agent hydrophobe (3) on parvient à ce que les matières premières actives (2) et par conséquent, les granulés formant le produit en question, soient stables dans le milieu ambiant et que le possible mauvais goût, odeur ou couleur des matières premières actives (2) soit camouflé par l'agent hydrophobe (3).

L'agent hydrophobe (3) peut être composé d'un des produits d'origine végétale mentionnés au point précédent ou d'un mélange de plusieurs d'eux, ayant différents points de fusion, de sorte que leur application sur les matières premières actives (2) se réalise à une température suffisamment basse pour assurer le maintien des propriétés intégrales desdites matières premières actives (2).

En tout cas, l'agent hydrophobe (3) utilisé peut avoir un point de fusion précis dans chaque application, selon la nature ou le type de matières premières actives (2) et selon le domaine d'application auquel est destiné le produit final.

Dans l'exemple de réalisation montré dans la figure 2, la particule ou granulé de produit (1) présente une composition similaire à la précédente (sous forme de matrices lipidiques) avec les matières premières actives (2) et l'agent hydrophobe (3), présentant de plus dans ce cas un pelliculage extérieur (4) d'un agent hydrophobe qui peut être le même que celui portant la référence (3) ou d'autres quelconques parmi ceux mentionnés précédemment.

Cette réalisation peut être utilisée dans les applications pour lesquelles il convient que l'agent ou agents hydrophobes formant le pelliculage extérieur (4) aient un point de fusion autre que celui de l'agent ou agents hydrophobes (3).

Le granulé de produit (1) représenté dans l'exemple de réalisation de la figure 3, constitue un cas particulier des précédents, étant constitué d'une matière première active (2), seule ou combinée à d'autres, et d'un agent hydrophobe (3) qui forme un pelliculage extérieur.

Les matières actives (2) utilisées dans les exemples de réalisation décrits peuvent appartenir, à titre d'exemple, à une famille chimique ou biologique quelconque, n'étant pas stables au milieu ambiant (à l'air, à la lumière et l'humidité) ou ayant de mauvaises caractéristiques organoleptiques et/ou s'il est intéressant qu'elles aient une réaction à température programmée dans le produit final en fonction de leur application.

A leur tour, l'agent ou agents hydrophobes (3) et ceux formant le pelliculage (4) peuvent être appliqués à l'aide de froid, vide, chaleur, forces de cisaillement, transformation en nodules, frittage, compactage, agglomération, transformation en globules ou combinaisons des précédents.

Comme on l'a déjà dit, l'agent hydrophobe, substantiellement insoluble dans l'eau, en état solide, peut être composé de produits d'origine végétale tels que cires d'utilisation alimentaire, graisses alimentaires ou margarines ou acides gras, ou des mono ou di ou triglycérides d'acides gras non polymérisés à chaîne linéaire saturés et insaturés, estérifiés ou pas, présents dans les huiles et/ou graisses alimentaires, ou des mono ou di ou triglycérides d'acides gras estérifiés avec les acides organiques alimentaires estérifiés ou pas et leurs sels calciques, de sodium, potassiques ou les esters de glycérol ou les esters de glycérol interestérifié avec des acides gras d'huile de ricin, ou des esters de sorbitan.

Une fois la nature de l'invention suffisamment décrite, ainsi qu'un exemple de réalisation préférentiel, il est constaté à l'effet opportun que les matières, forme, dimensions et disposition des éléments décrits pourront être modifiés à condition que cela ne suppose pas une altération des caractéristiques essentielles de l'invention qui sont revendiquées ci-après.

## Revendications

1. Améliorations introduites dans des produits destinés à la consommation humaine ou animale, **caractérisées en ce qu'**elles se présentent sous forme de granulés ou de particules constituées de matières actives seules, ou associées à d'autres, ou en combinaison à des excipients, et d'un agent hydrophobe substantiellement insoluble dans l'eau, en état solide, avec un point de fusion compris entre 10 et 80 degrés centigrades, en maintenant la matière active isolée et l'empêchant de commencer son activité tant que n'a pas eu lieu la fusion de l'agent hydrophobe.

2. Améliorations, selon la revendication 1, **caractérisées en ce que** le traitement des matières actives avec l'agent hydrophobe, peut être obtenu à l'aide de froid, vide, chaleur, forces de cisaillement, transformation en nodules, frittage, compactage, agglomération, transformation en globules ou combinaisons des précédents.

3. Améliorations, selon les revendications précédentes, **caractérisées en ce que** l'agent hydrophobe incorporé dans le produit est insoluble dans l'eau, en état solide, et soluble, miscible ou dispersable dans l'eau quand il est fondu.

4. Améliorations, selon les revendications précédentes, **caractérisées en ce que** la matière hydrophobe maintient les caractéristiques de protection de la matière première active, empêchant sa dégradation, réaction et libération de ses caractéristiques organoleptiques entre les -40 et +75 degrés centigrades.

5. Améliorations, selon les revendications précédentes, **caractérisées en ce que** la température de la matière première active, seule ou en combinaison avec d'autres matières premières actives ou des excipients, se maintient à une température inférieure à 50°C pendant l'incorporation du produit hydrophobe.

6. Améliorations, selon les revendications précédentes, **caractérisées en ce que** l'agent hydrophobe substantiellement insoluble dans l'eau, en état solide, est composé de produits d'origine végétale tels que : cires d'utilisation alimentaire, graisses alimentaires ou margarines ou préparations grasses telles que : graisses inertes ou acides gras, ou des mono ou di ou triglycérides d'acides gras non polymérisés à chaîne linéaire saturés et insaturés, estérifiés ou pas, présents dans les huiles et/ou graisses alimentaires, ou des mono ou di ou triglycérides d'acides gras estérifiés avec les acides organiques alimentaires estérifiés ou pas et leurs sels calciques, de sodium et potassiques, ou les esters de glycérol ou les esters de glycérol interestérifié avec des acides gras d'huile de ricin, ou des esters de sorbitan ou des combinaisons des précédents.

7. Améliorations, selon les revendications précédentes, **caractérisées en ce que** la quantité en poids d'agent hydrophobe incorporé, est compris entre 5% et 50% du poids du produit final.

8. Améliorations, selon les revendications précédentes, **caractérisées en ce que** les granulés ou particules composées de la matière première active, avec ou sans excipients, et de l'agent hydrophobe, présentent des dimensions comprises entre 50 microns et 10 millimètres.

9. Améliorations, selon les revendications précédentes, **caractérisées en ce que** la fusion ou dégradation de l'agent hydrophobe incorporé dans la matière active est seulement possible au moyen de procédés enzymatiques ou d'un point ou séquence de points de température.
